(19) Europäisches Patentamt
European Patent Office
Office européen des brevets

(11) Veröffentlichungsnummer : **0 032 103**
**B1**

(12) # EUROPÄISCHE PATENTSCHRIFT

(45) Veröffentlichungstag der Patentschrift : **13.06.84**

(21) Anmeldenummer : **80730081.9**

(22) Anmeldetag : **22.12.80**

(51) Int. Cl.³ : **C 07 J 31/00**, A 61 K 31/57// C07J5/00, C12P33/08

(54) **Neue Kortikoid-17-thioacetale, ihre Herstellung und Verwendung.**

(30) Priorität : **21.12.79 DE 2952003**

(43) Veröffentlichungstag der Anmeldung : **15.07.81 Patentblatt 81/28**

(45) Bekanntmachung des Hinweises auf die Patenterteilung : **13.06.84 Patentblatt 84/24**

(84) Benannte Vertragsstaaten : **AT BE CH DE FR GB IT LI LU NL SE**

(56) Entgegenhaltungen :
**EP-A- 0 003 341**
**EP-A- 0 003 519**
**Die Akte enthält technische Angaben, die nach dem Eingang der Anmeldung eingereicht wurden und die nicht in dieser Patentschrift enthalten sind.**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Annen, Klaus, Dr.**
**Seegefelder Strasse 194**
**D-1000 Berlin 20 (DE)**
Erfinder : **Laurent, Henry, Dr.**
**Glambecker Weg 21**
**D-1000 Berlin 28 (DE)**
Erfinder : **Hofmeister, Helmut, Dr.**
**Weislingenstrasse 4**
**D-1000 Berlin 28 (DE)**
Erfinder : **Petzoldt, Karl, Dr.**
**Flachsweg 10**
**D-1000 Berlin 38 (DE)**
Erfinder : **Wiechert, Rudolf, Prof.**
**Petzowerstrasse 8a**
**D-1000 Berlin 39 (DE)**
Erfinder : **Wendt, Hans, Dr.**
**Ernst-Ring-Strasse 14**
**D-1000 Berlin 38 (DE)**
Erfinder : **Kapp, Joachim-Friedrich, Dr.**
**Ludolfinger Weg 51**
**D-1000 Berlin 28 (DE)**

**EP 0 032 103 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft den in den Ansprüchen gekennzeichneten Gegenstand.

Die Kortikoid-17-thioacetale der allgemeinen Formel I gemäß Anspruch 1 können als Substituenten X ein Fluoratom, eine Methylgruppe oder ein Wasserstoffatom tragen. Bevorzugt sind solche Kortikoid-17-thioacetale, die als Substituenten X eine Methylgruppe oder insbesondere ein Wasserstoffatom tragen.

Als Substituenten $R_1$ können die Kortikoid-17-thioacetale ein Wasserstoffatom oder eine niedere Alkylgruppe tragen. Unter einer niederen Alkylgruppe soll vorzugsweise eine Gruppe mit 1 bis 4 Kohlenstoffatomen verstanden werden, wie zum Beispiel die Methylgruppe, die Äthylgruppe, die Propylgruppe, die Isopropylgruppe oder die Butylgruppe. Besonders bevorzugt sind solche Kortikoid-17-thioacetale der allgemeinen Formel I, die als Substituenten $R_1$ ein Wasserstoffatom tragen.

Die Kortikoid-17-thioacetale der allgemeinen Formel I gemäß Anspruch 1 können als Substituenten $R_2$ ein Wasserstoffatom oder eine Acylgruppe tragen. Unter einer Acylgruppe $R_2$ soll eine Gruppe verstanden werden, welche man bei Kortikoiden üblicherweise als Estergruppe in der 21-Position des Moleküls verwendet. Geeignete Acylgruppen sind beispielsweise Gruppen mit 1 bis 16 Kohlenstoffatomen, die sich von geradkettigen oder verzweigten, gesättigten oder ungesättigten aliphatischen Mono- oder Dicarbonsäuren ableiten, welche in üblicher Weise, beispielsweise durch Hydroxygruppen, Aminogruppen oder Halogenatome substituiert sein können.

Ferner eignen sich als Acylgruppen auch Reste cycloaliphatischer, aromatischer, gemischt aromatisch-aliphatischer oder heterocyclischer Säuren, die ebenfalls in üblicher Weise substituiert sein können. Als geeignete Acylgruppen seien beispielsweise genannt :

Die Formyl-, Acetyl-, Propionyl-, Butyryl-, Pentanoyl-, Hexanoyl-, Octanoyl-, Undecanoyl-, Dimethylacetyl-, Trimethylacetyl-, Diäthylacetyl-, tert.-Butylacetyl-, Benzoyl-, Phenacetyl-, Cyclopentylpropionyl-, Hydroxyacetyl-, Monochloracetyl-, Dichloracetyl-, Trichloracetyl-, ferner die Dimethylaminoacetyl-, die Trimethylaminoacetyl-, die Diäthylaminoacetyl-, die Piperidinoacetyl-, die Nicotinoyl-, die ω-Carboxypropionyl- und die ω-Carboxypentanoylgruppe.

Bevorzugte Acylgruppen $R_2$ sind solche, die 1 bis 8 Kohlenstoffatome besitzen und insbesondere solche, die sich von einer 1 bis 6 Kohlenstoffatome enthaltenden geradkettigen oder verzweigten Alkancarbonsäure ableiten.

Aus der europäischen Patentanmeldung 0 003 341 sind Kortikoid-17-acetale und Kortikoid-17-thioacetale vorbekannt. Die erfindungsgemäßen Kortikoid-17-thioacetale haben gegenüber den vorbekannten strukturanalogen Verbindungen den Vorzug, daß sie bei topischer Applikation eine stärkere antiinflammatorische Wirksamkeit entfalten und/oder daß sie eine günstigere Dissoziation zwischen erwünschter topischer Wirksamkeit und unerwünschter systemischer Wirkung besitzen.

Die topische Wirksamkeit kann mit Hilfe des Vasokonstriktionstestes wie folgt bestimmt werden.

Der Test wird an je 8 gesunden Probanden beider Geschlechter durchgeführt, die in den letzten zwei Wochen keine lokale Corticosteroidbehandlung erhalten hatten. Nach dem Entfernen des Stratum corneum bis zum Stratum lucidum auf dem Rücken der Probanden (20-40 Tesafilm-Abrisse) werden je 0,1 g der Zubereitungen auf 4 $cm^2$ große Felder ohne Okkulsionsverband aufgetragen. Um zu vermeiden, daß die gleiche Zubereitung jeweils auf identische Hautareale appliziert wird, wird in rotierender Folge aufgetragen.

Die Vasokonstriktion wird visuell nach 4 und 8 Stunden durch den Prüfer nach folgenden Wirkungsgraden beurteilt : 1 = absolute Abblassung, 2 = geringes Resterythem, 3 = mittelgradiges Erythem, Rötungsintensität im mittleren Bereich von gestrippter, unbehandelter und nicht geschädigter Haut, 4 = Erythem mit geringen Aufhellungen, 5 = keine Abblassung oder Verstärkung des Erythems.

Die Einzelbeurteilungen werden gemittelt.

In jeder Versuchsreihe wird als Referenzsubstanz das Diflucortolon-21-valerianat (= 6α, 9α-Difluor-11β-hydroxy-16α-methyl-21-valeryloxy-1,4-pregnadien-3,20-dion = DFV) verwendet.

Es wird jeweils die Differenz Δ der in den einzelnen Untersuchungsreihen ermittelten mittleren Wirkungsgraden von DFV und Testsubstanz ermittelt. Positive Abweichungen Δ zeigen eine günstigere, negative Abweichungen zeigen eine ungünstigere Beurteilung der Testsubstanz im Vergleich zu DFV an.

In der nachfolgenden Tabelle sind die beobachteten Testergebnisse aufgeführt, die bei der Behandlung der Probanden mit einer 0,1 ppm Wirkstoff enthaltenden Zubereitung erzielt wird.

Die systemische Wirksamkeit der Verbindungen kann mit Hilfe des bekannten Hautreißversuchs wie folgt ermittelt werden :

Männliche Wistar-Ratten (Gewicht 150 ± 20 g) wird 6 mg Testsubstanz täglich in 0,2 ml Cetiol über 20 Tage auf die von Haaren befreite Rückenhaut appliziert. Eine besondere Plastikabdeckvorrichtung verhindert, daß die Tiere die aufgetragene Substanz oral aufnehmen. Während des Versuchs wird das Körpergewicht kontrolliert und bei Versuchsende das Organgewicht der Milz bestimmt. Die behandelte Rückenhaut wird in einer Größe von etwa 5 × 5 cm abpräpariert. Die Hautdicke wird bestimmt. Aus dem Hautstück werden mit Hilfe einer Stahlstanze zwei doppel-T-förmige Streifen herausgeschnitten. Der Hautstreifen wird mit seinen breiten Enden in die Zugbacken eines speziellen Meßgerätes eingespannt. Es wird dann die Kraft bestimmt, die zum Dehnen und zum Zerreißen des Hautstreifens notwendig ist.

Die nachfolgende Tabelle zeigt die in diesem Versuch erhaltenen Testergebnisse.

Tabelle

| Nr. | Substanz | Vasokonstriktionstest Δ nach 4 Stdn. | nach 8 Stdn. | Hautreißversuch Gewicht der Tiere | Milzgewicht | Reißfestigkeit der Haut | Maximaler Verformungswiderstand der Haut |
|---|---|---|---|---|---|---|---|
| I | 11ß,21-Dihydroxy-17α-methoxymethoxy-4-pregnen-3,20-dion (Europ. Patentanm. Nr. 3341) | +0,3 | +0,7 | -14 % | -28 % | -36 % | - 43 % |
| II | 11ß,21-Dihydroxy-17α-methylthiomethoxy-4-pregnen-3,20-dion | +0,9 | +0,9 | -7 % | -15 % | -7 % | - 8 % |

Die neuen Kortikoid-17-thioacetale eignen sich in Kombination mit den in der galenischen Pharmazie üblichen Trägermitteln zur lokalen Behandlung von Kontaktdermatitis, Ekzemen der verschiedensten Art, Neurodermatosen, Erythrodermie, Verbrennungen, Pruritis vulvae et ani, Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wirkstoffe mit geeigneten Zusätzen in die gewünschte Applikationsform, wie zum Beispiel : Lösungen, Lotionen, Salben, Cremen oder Pflaster, überführt. In den so formulierten Arzneimitteln ist die Wirkstoffkonzentration von der Applikationsform abhängig. Bei Lotionen und Salben wird vorzugsweise eine Wirkstoffkonzentration von 0,001 % bis 1 % verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege, wie zum Beispiel des Bronchialasthmas oder der Rhinitis verwendet werden können.

Ferner eignen sich die neuen Kortikoide auch in Form von Kapseln, Tabletten oder Dragees, die vorzugsweise 10-200 mg Wirkstoff enthalten und oral appliziert werden oder in Form von Suspensionen, die vorzugsweise 100-500 mg Wirkstoff pro Dosiseinheit enthalten und rektal appliziert werden auch zur Behandlung allergischer Erkrankungen des Darmtraktes, wie der Kolitis ulcerosa und der Kolitis granulomatosa.

Die neuen Kortikoid-17-thioacetale können nach dem im Anspruch 13 gekennzeichneten Verfahren hergestellt werden, welches unter den Bedingungen durchgeführt werden kann, wie sie in der europäischen Patentanmeldung 0 003 541 beschrieben sind. Die nachfolgenden Ausführungsbeispiele dienen zur Erläuterung des erfindungsgemäßen Verfahrens.

Beispiel 1

21-Acetoxy-11β-hydroxy-17-methylthiomethoxy-4-pregnen-3,20-dion

a) Eine Lösung von 5,0 g Hydrocortisonacetat in 25 ml Pyridin wird bei − 15 °C tropfenweise mit 3 ml Trifluoressigsäureanhydrid versetzt und 10 Minuten bei − 10 °C gerührt. Man gibt auf eine Eiswasser-Kochsalzlösung und filtriert den Niederschlag ab. Den Rückstand nimmt man in Methylenchlorid auf, wäscht neutral und engt nach dem Trocknen über Natriumsulfit im Vakuum ein. Ausbeute 5,1 g 21-Acetoxy-17α-hydroxy-11β-trifluoracetoxy-4-pregnen-3,20-dion.

b) 3,7 g des obigen Rohproduktes werden über Nacht in einem Gemisch aus 31 ml Dimethylsulfoxid, 20,5 ml Essigsäureanhydrid und 6,2 ml Eisessig bei Raumtemperatur gerührt. Die Reaktionslösung wird auf eine Ioproz. Natriumcarbonat-Lösung gegeben und der Niederschlag abfiltriert. Man löst den Rückstand in Methylenchlorid und arbeitet nach dem Neutralwaschen wie üblich auf. Nach der Chromatographie an 600 mg Kieselgel mit einem Methylenchlorid-Aceton-Gradienten (0-12 % Aceton) isoliert man 3,48 g 21-Acetoxy-17α-methylthiomethoxy-11β-trifluoracetoxy-4-pregnen-3,20-dion.

c) 2,0 g 21-Acetoxy-17α-methylthiomethoxy-11β-trifluoracetoxy-4-pregnen-3,20-dion werden in 50 ml Methanol und 2,5 ml Triethylamin 4 Stunden bei Raumtemperatur gerührt. Man reinigt das Rohprodukt an 300 g Kieselgel mit einem Methylenchlorid-Aceton-Gradienten (0-8 % Aceton) und isoliert 1,54 g 21-Acetoxy-11β-hydroxy-17α-methylthiomethoxy-4-pregnen-3,20-dion.

Schmelzpunkt 167 °C.

$[\alpha]_D^{25}$ = 160° (Chloroform).

UV : $\varepsilon_{241}$ = 16 100 (Methanol).

Beispiel 2

11β, 21-Dihydroxy-17-methylthiomethoxy-4-pregnen-3,20-dion

Man rührt 2,4 g 21-Acetoxy-17α-methylthiomethoxy-11β-trifluoracetoxy-4-pregnen-3,20-dion in 50 ml Methanol und 3,0 ml Triethylamin über Nacht bei Raumtemperatur. Das Rohprodukt wird an 350 g Kieselgel mit einem Methylenchlorid-Aceton-Gradienten (0-12 % Aceton) gereinigt. Ausbeute : 1,25 g 11β, 21-Dihydroxy-17-methylthiomethoxy-4-pregnen-3,20-dion.

Schmelzpunkt 148 °C.

$[\alpha]_D^{25}$ = + 172° (Chloroform).

UV : $\varepsilon_{242}$ = 16 200 (Methanol).

Beispiel 3

11β-Hydroxy-17α-methylthiomethoxy-21-propionyloxy-4-pregnen-3,20-dion

Ausgehend von 3,5 g 11β, 17α-Dihydroxy-21-propionyloxy-4-pregnen-3,20-dion erhält man analog der

Reaktionsfolge von Ia-c 920 mg 11β-Hydroxy-17α-methylthiomethoxy-21-propionyloxy-4-pregnen-3,20-dion.

Schmelzpunkt 142 °C.
$[\alpha]_D^{25}$ = + 167° (Pyridin).
UV : $\varepsilon_{241}$ = 16 100 (Methanol).

Beispiel 4

21-Butyryloxy-11β-hydroxy-17α-methylthiomethoxy-4-pregnen-3,20-dion

Analog der Reaktionsfolge 1a-1c erhält man aus 2,8 g 21-Butyryloxy-11β-17α-dihydroxy-4-pregnen-3,20-dion 780 mg 21-Butyryloxy-11β-hydroxy-17α-methylthiomethoxy-4-pregnen-3,20-dion.

Schmelzpunkt 133 °C.
$[\alpha]_D^{25}$ = + 160 °C (Pyridin).
UV : $\varepsilon_{242}$ = 16 300 (Methanol).

Beispiel 5

11β-Hydroxy-17α-methylthiomethoxy-21-valeryloxy-4-pregnen-3,20-dion

1,2 g 11β, 21-Dihydroxy-17α-methylthiomethoxy-4-pregnen-3,20-dion werden in 12 ml Pyridin und 6 ml n-Valeriansäureanhydrid 17 Stunden bei Raumtemperatur gerührt. Nach der üblichen Aufarbeitung und Reinigung an 150 g Kieselgel mit einem Methylenchlorid-Aceton-Gradienten (0-12 % Aceton) isoliert man 987 mg 11β-Hydroxy-17α-methylthio-methoxy-21-valeryloxy-4-pregnen-3,20-dion.

Schmelzpunkt 138 °C.
$[\alpha]_D^{25}$ = + 157° (Pyridin).
UV : $\varepsilon_{241}$ = 16 100 (Methanol).

Beispiel 6

11β-Hydroxy-17α-methylthiomethoxy-21-trimethylacetoxy-4-pregnen-3,20-dion

Eine Lösung von 1,6 g 11β, 21-Dihydroxy-17α-methylthiomethoxy-4-pregnen-3,20-dion in 15 ml Pyridin und 8 ml Trimethylessigsäureanhydrid wird über Nacht bei Raumtemperatur gerührt. Man arbeitet wie üblich auf und isoliert nach der chromatographischen Reinigung 1,1 g 11β-Hydroxy-17α-methylthiomethoxy-21-trimethylacetoxy-4-pregnen-3,20-dion.

Schmelzpunkt 175 °C.
$[\alpha]_D^{25}$ = + 158° (Pyridin).
UV : $\varepsilon_{242}$ = 16 200 (Methanol).

Beispiel 7

21-Acetoxy-11β-hydroxy-6α-methyl-17α-methylthiomethoxy-4-pregnen-3,20-dion

4,1 g 21-Acetoxy-11β, 17α-dihydroxy-6α-methyl-4-pregnen-3,20-dion werden unter den Bedingungen der Reaktionsfolge 1a-1c zu 1,7 g 21-Acetoxy-11β-hydroxy-6α-methyl-17α-methylthiomethoxy-4-pregnen-3,20-dion, umgesetzt.

Schmelzpunkt 189 °C.

Beispiel 8

Ein 1L-Erlenmeyerkolben, der 200 ml einer 30 Minuten bei 120 °C im Autoklaven sterilisierten Nährlösung aus 3 % Glukose, 1 % Corn steep, 0,2 % $NaNO_3$, 0,1 % $KH_2PO_4$, 0,2 % $K_2HPO_4$, 0,05 % $MgSO_4 \cdot 7 H_2O$, 0,002 % $FeSO_4 \cdot 7 H_2O$ und 0,05 % KCl enthält, wird mit einer Lyophilkultur von Cuvularia lunata (NRRL 2 380) beimpft und 70 Stunden bei 30 °C auf einem Rotationsschüttler bewegt. Mit jeweils 50 ml dieser Anzuchtskultur werden zwei 2 L-Erlenmeyerkolben beimpft, die mit je 500 ml sterilisiertem Nährmedium der gleichen Zusammensetzung wie die vorkultur gefüllt ist. Nach einer Anwachszeit von 6 Stunden bei 30 °C auf der Schüttelmaschine gibt man unter sterilen Bedingungen in jeden Kolben 100 mg 21-Acetoxy-17α-methylthiomethoxy-4-pregnen-3,20-dion, gelöst in 0,5 ml Dimethylformamid, hinzu und läßt weiter schütteln. Der Verlauf der Fermentation wird durch Entnahme von Proben kontrolliert, die mittels Methylisobutylketon extrahiert und dünnschichtchromatographisch analysiert werden.

Nach 64 Stunden Kontaktzeit ist die Umsetzung des Substrates beendet. Die beiden Kolbeninhalte werden vereinigt, das Mycel abfiltriert und das Kulturfiltrat mit Methylisobutylketon extrahiert. Den Extrakt dampft man im Vakuum zur Trockne ein und chromatographiert den Rückstand über eine Kieselgelsäule mittels des Lösungsmittelgradienten Methylenchlorid-Methylenchlorid/Aceton 7 + 3. Die

Hauptfraktion wird zur Trockne eingeengt und aus Aceton/Diisopropylether kristallisiert. Man erhält 148 mg 11β, 21-Dihydroxy-17α-methylthiomethoxy-4-pregnen-3,20-dion vom Schmelzpunkt 149 °C.

Zur Herstellung des Ausgangsmaterials werden 1,5 g 21-Acetoxy-17α-hydroxy-4-pregnen-3,20-dion analog Beispiel 1 b umgesetzt und aufgearbeitet. Nach der Chromatographie an 130 g Kieselgel mit einem Methylenchlorid-Aceton-Gradienten (0-12 % Aceton) isoliert man 1,28 g 21-Acetoxy-17α-methylthiomethoxy-4-pregnen-3,20-dion vom Schmelzpunkt 149 °C.

**Ansprüche**

1. Kortikoid-17-thioacetale der allgemeinen Formel I

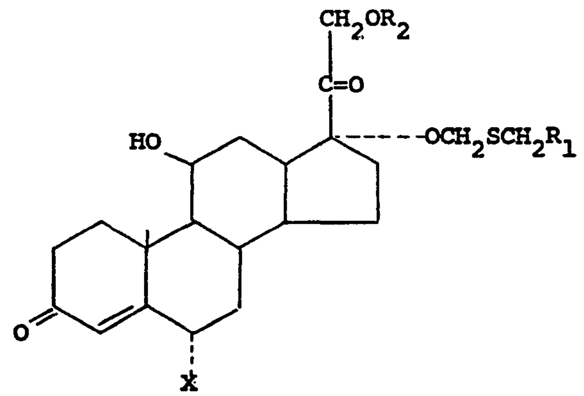

(I)

worin

X ein Wasserstoffatom, ein Fluoratom oder eine Methylgruppe,

$R_1$ ein Wasserstoffatom oder eine niedere Alkylgruppe und

$R_2$ ein Wasserstoffatom oder eine Acylgruppe bedeuten.

2. Kortikoid-17-thioacetale der allgemeinen Formel I gemäß Anspruch 1, worin $R_1$ ein Wasserstoffatom bedeutet.

3. Kortikoid-17-thioacetale der allgemeinen Formel I gemäß Anspruch 1 und 2, worin $R_2$ ein Wasserstoffatom oder eine 1 bis 8 Kohlenstoffatome enthaltende Acylgruppe bedeutet.

4. Kortikoid-17-thioacetale der allgemeinen Formel I gemäß Anspruch 1 bis 3, worin X ein Wasserstoffatom bedeutet.

5. 21-Acetoxy-11β-hydroxy-17α-methylthiomethoxy-4-pregnen-3,20-dion.

6. 11β, 21-Dihydroxy-17α-methylthiomethoxy-4-pregnen-3,20-dion.

7. 11β-Hydroxy-17α-methylthiomethoxy-21-propionyloxy-4-pregnen-3,20-dion.

8. 21-Butyryloxy-11β-hydroxy-17α-methylthiomethoxy-4-pregnen-3,20-dion.

9. 11β-Hydroxy-17α-methylthiomethoxy-21-valeryloxy-4-pregnen-3,20-dion.

10. 11β-Hydroxy-17α-methylthiomethoxy-21-trimethylacetoxy-4-pregnen-3,20-dion.

11. 21-Acetoxy-11β-hydroxy-6α-methyl-17α-methylthiomethoxy-4-pregnen-3,20-dion.

12. Pharmazeutische Präparate enthaltend ein oder zwei Kortikoid-17-thioacetale gemäß Anspruch 1 bis 11.

13. Verfahren zur Herstellung von Kortikoid-17-thioacetalen der allgemeinen Formel I gemäß Anspruch 1, dadurch gekennzeichnet, daß man in an sich bekannter Weise

a) ein Kortikoid der allgemeinen Formel II,

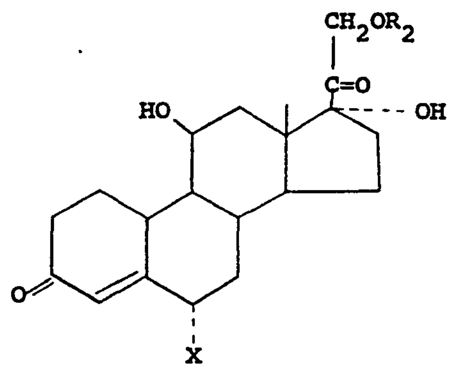

(II)

worin X und $R_2$ die im Anspruch 1 genannte Bedeutung besitzen, gegebenenfalls, nach intermediärem Schutz der 11- und 21-Hydroxygruppe mit einem Sulfoxid der allgemeinen Formel III

$CH_3SOCH_2R_1$ (III)

worin $R_1$ die im Anspruch 1 genannte Bedeutung besitzt, umsetzt und gegebenenfalls eine vorhandene 21-Estergruppe verseift oder eine 21-Hydroxygruppe verestert, oder

b) ein Steroid-17-thioacetal der allgemeinen Formel IV

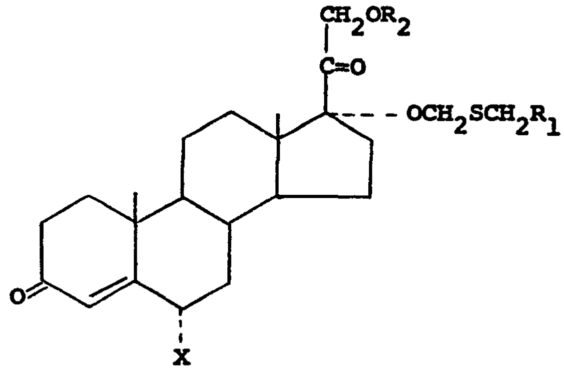

(IV)

worin X, $R_1$ und $R_2$ die im Anspruch 1 genannte Bedeutung besitzen, gegebenenfalls unter gleichzeitiger Abspaltung einer vorhandenen 21-Estergruppe mit einer zur 11β-Hydroxylierung befähigten Mikroorganismen-Kultur fermentiert und gegebenenfalls vorhandene 21-Hydroxy-gruppen verestert.

**Claims**

1. Corticoid-17-thioacetals of the general formula

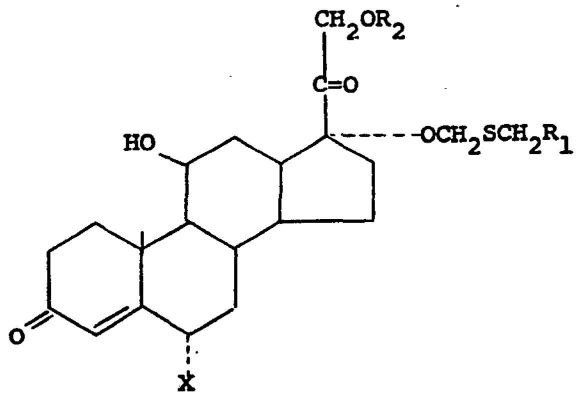

(I)

in which

X represents a hydrogen atom, a fluorine atom or a methyl group,

$R_1$ represents a hydrogen atom or a lower alkyl group, and

$R_2$ represents a hydrogen atom or an acyl group.

2. Corticoid-17-thioacetals of the general formula I according to claim 1, in which $R_1$ represents a hydrogen atom.

3. Corticoid-17-thioacetals of the general formula I according to claims 1 and 2 in which $R_2$ represents a hydrogen atom or an acyl group containing from 1 to 8 carbon atoms.

4. Corticoid-17-thioacetals of the general formula I according to claims 1 to 3 in which X represents a hydrogen atom.

5. 21-acetoxy-11β-hydroxy-17α-methylthiomethoxy-4-pregnen-3,20-dione.

6. 11β, 21-dihydroxy-17α-methylthiomethoxy-4-pregnen-3,20-dione.

7. 11β-hydroxy-17α-methylthiomethoxy-21-propionyloxy-4-pregnen-3,20-dione.

8. 21-butyryloxy-11β-hydroxy-17α-methylthiomethoxy-4-pregnen-3,20-dione.

9. 11β-hydroxy-17α-methylthiomethoxy-21-valeryloxy-4-pregnen-3,20-dione.

10. 11β-hydroxy-17α-methylthiomethoxy-21-trimethyl-acetoxy-4-pregnen-3,20-dione.

11. 21-acetoxy-11β-hydroxy-6α-methyl-17α-methylthiomethoxy-4-pregnen-3,20-dione.

12. Pharmaceutical preparations containing one or two corticoid-17-thioacetals according to claims 1 to 11.

13. Process for the manufacture of corticoid-17-thioacetals of the general formula I according to claim 1, characterised in that, in a manner known *per se*,

a) a corticoid of the general formula II,

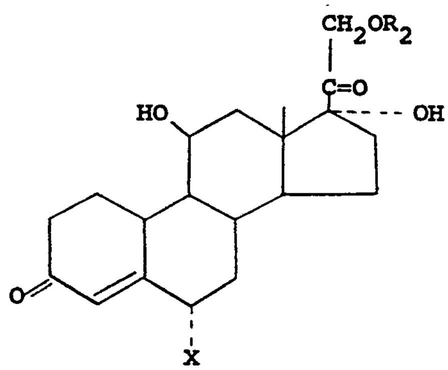

(II)

in which X and $R_2$ have the meanings given in claim 1, is reacted, optionally after intermediate protection of the 11- and 21-hydroxy groups, with a sulphoxide of the general formula III

$$CH_3SOCH_2R_1 \quad (III)$$

in which $R_1$ has the meaning given in claim 1 and, optionally, a 21-ester group present is hydrolysed or a 21-hydroxy group is esterified, or

b) a steroid-17-thioacetal of the general formula

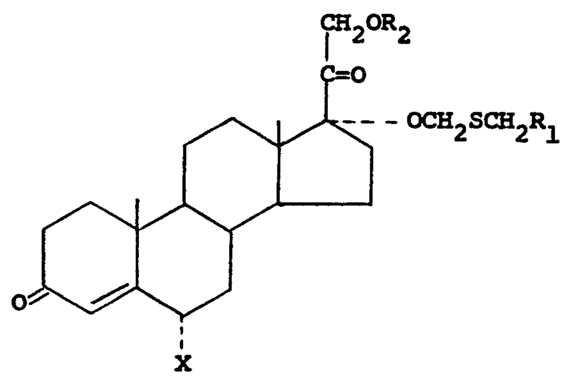

(IV)

in which X, $R_1$ and $R_2$ have the meanings given in claim 1 is fermented, optionally with simultaneous splitting off of a 21-ester group present, with a microorganism culture that is capable of 11β-hydroxylation and 21-hydroxy groups optionally present are esterified.

**Revendications**

1. Thio-acétals en 17 de corticostéroïdes qui répondent à la formule générale (I)

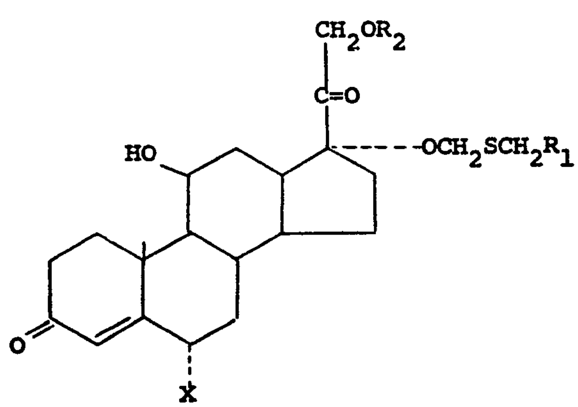

(I)

dans laquelle

X représente un atome d'hydrogène ou, de fluor ou le radical méthyle,

$R_1$ représente un atome d'hydrogène ou un radical alkyle inférieur, et

$R_2$ représente un atome d'hydrogène ou un radical acyle.

2. Thio-acétals en 17 de corticostéroïdes de formule générale (I) selon la revendication 1, dans lesquels $R_1$ représente un atome d'hydrogène.

3. Thio-acétals en 17 de corticostéroïdes de formule générale (I) selon l'une des revendications 1 et 2, dans lesquels $R_2$ représente un atome d'hydrogène ou un radical acyle contenant de 1 à 8 atomes de carbone.

4. Thio-acétals en 17 de corticostéroïdes de formule générale (I) selon l'une quelconque des revendications 1 à 3, dans lesquels X représente un atome d'hydrogène.

5. Acétoxy-21 hydroxy-11β méthylthiométhoxy-17α prégnène-4 dione-3,20.

6. Dihydroxy-11β, 21 méthylthiométhoxy-17α prégnène-4 dione-3,20.

7. Hydroxy-11β méthylthiométhoxy-17α propionyloxy-21 prégnène-4 dione-3,20.

8. Butyryloxy-21 hydroxy-11β méthylthiométhoxy-17α prégnène-4 dione-3,20.

9. Hydroxy-11β méthylthiométhoxy-17α valéryloxy-21 prégnène-4 dione-3,20.

10. Hydroxy-11β méthylthiométhoxy-17α triméthylacétoxy-21 prégnène-4 dione-3,20.

11. Acétoxy-21 hydroxy-11β méthyl-6α méthylthiométhoxy-17α prégnène-4 dione-3,20.

12. Médicaments renfermant un ou deux thio-acétals en 17 de corticostéroïdes selon l'une quelconque des revendications 1 à 11.

13. Procédé de préparation de thio-acétals en 17 de corticostéroïdes de formule générale (I) selon la revendication 1, procédé caractérisé en ce que, en opérant de manière connue :

a) on fait réagir un corticostéroïde répondant à la formule générale (II)

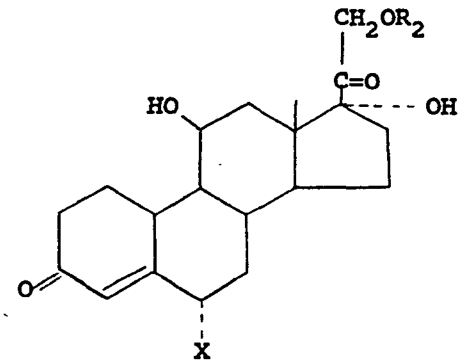

(II)

dans laquelle X et $R_2$ ont les significations données à la revendication 1, éventuellement après avoir protégé intermédiairement les radicaux hydroxy en 11 et en 21, avec un sulfoxyde répondant à la formule générale (III)

$$CH_3SOCH_2R_1 \qquad \text{(III)}$$

dans laquelle $R_1$ a la signification donnée à la revendication 1, et éventuellement on saponifie un radical ester en 21 s'il y en a un ou on estérifie un radical hydroxy en 21, ou

b) on fait fermenter un thio-acétal en 17 de stéroïde répondant à la formule générale (IV)

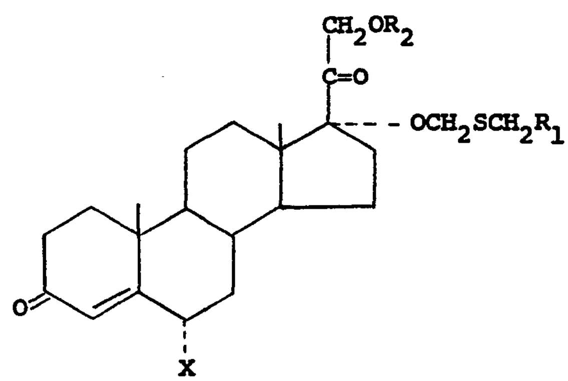

(IV)

dans laquelle X, $R_1$ et $R_2$ ont les significations données à la revendication 1, éventuellement avec coupure simultanée d'un éventuel radical ester en 21, avec une culture de micro-organismes capables d'effectuer une hydroxylation en 11β, et éventuellement on estérifie un radical hydroxy en 21 s'il y en a un.